# EUROPEAN PATENT APPLICATION

(11) **EP 0 719 553 A1**
(43) Date of publication of application: **03.07.1996**
(21) Application number: 95120569.9
(22) Date of filing: 27.12.1995
(51) Int. Cl.: A61K 31/275

(54) **Pharmaceutical composition for topical administration to the eye for treating allergic conjunctivitis**

(30) Priority: 26.12.1994 JP 322903/94
(71) Applicant: Senju Pharmaceutical Co., Ltd., Osaka-shi, Osaka 541 (JP); Toyo Boseki Kabushiki Kaisha, Osaka-shi Osaka 530 (JP)
(72) Inventor: Ogawa, Takahiro, Hyogo 662 (JP); Terai, Tadashi, Kobe-shi Hyogo 657 (JP); Haruna, Ken-ichi, Hyogo 670 (JP); Watanabe, Akihiko c/o Toyo Boseki K.K., Ohtsu-shi Shiga 520-02 (JP); Tominaga, Takanari c/o Toyo Boseki K.K., Ohtsu-shi Shiga 520-02 (JP); Taguchi, Hiroaki c/o Toyo Boseki K.K., Ohtsu-shi Shiga 520-02 (JP)
(74) Representative: VOSSIUS & PARTNER

(57) **Abstract**

A pharmaceutical composition for topical administration to the eye for treating allergic conjunctivitis, which comprises, as an active ingredient, the amide compound of the formula (I) 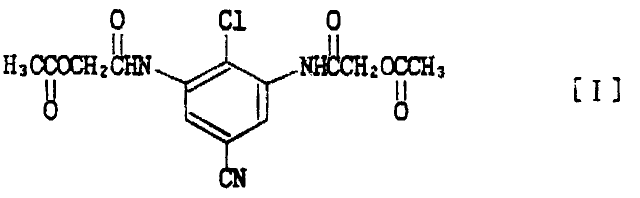
a method for treating allergic conjunctivitis comprising topical administration of this compound to the eye, use of this compound for topical administration to the eye for treatment of allergic conjunctivitis, and use of this compound for preparing a pharmaceutical composition for topical administration to the eye for treating allergic conjunctivitis. The above-mentioned amide compound exhibits antiallergic action when applied topically to the eye and is useful for the treatment of allergic conjunctivitis. The present invention provides a novel pharmaceutical use of this compound.

## Description

The present invention relates to a pharmaceutical composition for topical administration to the eye for treating allergic conjunctivitis, which comprises the amide compound of the formula (I) below as an active ingredient, a method for treating allergic conjunctivitis comprising topical administration of said compound to the eye, use of said compound for topical administration to the eye for treatment of allergic conjunctivitis, and to the use of said compound for preparing a pharmaceutical composition for topical administration to the eye for treating allergic conjunctivitis.

US Patent No. 4912135 (EP-A-315112) teaches that specific amide compounds inclusive of the compound of the formula (I) below [hereinafter to be referred to as compound (I)] exhibit superior antiallergic action by oral administration and strongly suppress immediate allergy, and that the compounds are useful for prophylaxis and treatment of bronchial asthma, hives and allergic rhinitis which are all classified under immediate allergy. Yet, no reference has been found that describes the topical application of said amide compound to the eye and the pharmacological action thereof.

Accordingly, there has been a demand for the development of a novel use of said amide compound.

It is therefore an object of the present invention to provide a new pharmaceutical use of compound (I) which belongs to said amide compound.

A compound which is intended for topical administration to the eye is required to show absorption to the eye and no detrimental effects such as irritation caused by topical administration of the compound to the eye, since the eye is a very sensitive organ as compared to other organs.

According to the present invention, it has now been found that compound (I) of the following formula (I) 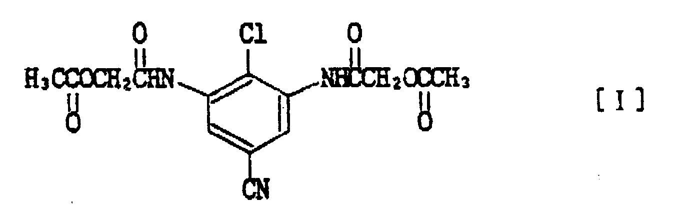
exhibits, when administered topically to an eye, superior therapeutic effects against allergic conjunctivitis and that compound (I) can be administered safely to the eye, in particular, with high safety to the anterior segment of the eye. In other words, it has been found that compound (I) can be applied topically to the eye and is extremely useful for treatment of allergic conjunctivitis.

It has also been found that compound (I) can be used effectively and safely for treatment of allergic conjunctivitis in the dose of 0.0005-8 mg/day.

It has been found, moreover, that the preferable concentration of compound (I) in an eyedrop is 0.001-2% (w/v).

That is, the present invention provides the following.
(1) A pharmaceutical composition for topical administration to the eye for treating allergic conjunctivitis, which comprises compound (I) as an active ingredient.
(2) Use of compound (I) for topical administration to the eye for treatment of allergic conjunctivitis.
(3) Use of compound (I) for preparing a pharmaceutical composition for topical administration to the eye for treatment of allergic conjunctivitis.
(4) A method for treating allergic conjunctivitis, comprising topically administering a pharmaceutically effective amount of compound (I) to the eye.

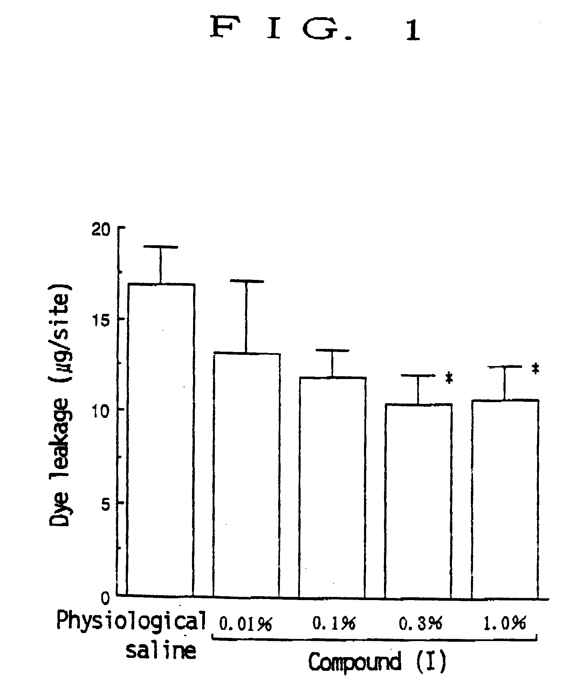

Fig. 1 is a graph showing the results of Experimental Example 1, wherein each value is mean±standard error (n=8-20) and * means the presence of significant difference (p<0.05) from control.

Compound (I)is a known compound and can be prepared, for example, by the method described in US Patent No. 4912135 (EP-A-315112).

The composition of the present invention can be formulated into various dosage forms generally employed for ophthalmic agents, such as eyedrops (e.g. ophthalmic solution, specifically ophthalmic aqueous suspension), with preference given to an ophthalmic aqueous suspension. Alternatively, the composition of the invention may be formulated into a solid preparation such as powder, granule and tablet, which can be dissolved or suspended in purified water or other medium.

Such preparation can be produced by a conventional method. The preparation is generally formulated by dissolving or suspending compound (I) in water (e.g. distilled water, sterile purified water and physiological saline) or non-aqueous solvent (e.g. non-aqueous solvent for injection).

The composition of the present invention may contain known additives such as buffer, isotonizing agent, preservative, stabilizer, thickener, chelating agent, pH regulator, solubilizing agent, surfactant and suspending agent, and other pharmaceutical products.

Examples of buffer include acetate buffers such as sodium acetate buffer, phosphate buffers such as sodium dihydrogenphosphate buffer, disodium hydrogenphosphate buffer, potassium dihydrogenphosphate buffer and dipotassium hydrogenphosphate buffer, amino acid salt buffers such as ε-aminocaproic acid buffer and sodium glutamate buffer, and boric acid buffer and borate buffer. Example of isotonizing agent include sodium chloride, glycerol, glucose, mannitol and sorbitol. Examples of prcocrvativo include quaternary ammonium salts such as benzalkonium chloride and benzethonium chloride, p-hydroxybenzoates such as methyl p-hydroxybenzoate, ethyl p-hydroxybenzoate, propyl p-hydroxybenzoate and butyl p-hydroxybenzoate, alcohols such as chlorobutanol and benzyl alcohol, and sodium dehydroacetate. Examples of stabilizer include ascorbic acid, cyclodextrin and sodium edetate. Examples of thickener include methyl cellulose and sodium chondroitin sulfate. Examples of chelating agent include sodium edetate and sodium citrate. Examples of pH regulator include hydrochloric acid, acetic acid, metaphosphoric acid and sodium hydroxide. Examples of solubilizing agent include propylene glycol, hydroxypropylmethylcellulose and polyvinyl alcohol. Examples of surfactant include polysorbate 80, polyoxyethylene hydrogenated castor oil (e.g. HCO60 manufactured by NOF Corporation) and benzalkonium chloride. Examples of suspending agent include hydroxypropylmethylcellulose, polyvinylpyrrolidone, polyvinyl alcohol, polysorbate 80 and polyoxyethylene hydrogenated castor oil.

The composition of the present invention may contain other drugs having a therapeutic effect against allergic conjunctivitis.

In the present invention, compound (I) is administered, for example, in a dose of about 0.0005-8 mg, preferably about 0.0025-4 mg, more preferably about 0.15-4 mg daily to an adult. While the content of compound (I) in a preparation varies depending on the dosage form, it is about 0.001-2% (w/v), preferably about 0.005-1% (w/v), more preferably about 0.3-1% (w/v) for an eyedrop. In case where a solid preparation is dissolved when in use, the concentration of the compound (I) is the same as that in the case of an eyedrop. For example, the compound (I) is administered to an adult by instilling one or two drops (e.g. 50-100 µl) of an eyedrop per administration 1-4 times a day.

The present invention is detailedly described in the following by illustrative Experimental Examples and Examples, to which the present invention is not limited.

### Experimental Example 1 : Effect on allergic conjunctivitis in rats

Male Wistar rats weighing about 100 g were used as test animals. An anti-ovalbumin serum diluted 32-fold with physiological saline (50 µl) was injected to a rat from under the upper eye lid conjunctiva for passive sensitization. Seventy-two hours from the injection, a mixed solution (0.5 ml) or equivalent amounts of 1% ovalbumin and 1% Evans blue was injected via a tail vein to topically induce passive anaphylaxis in conjunctiva. Thirty minutes thereafter, dye-leakage site in the eye lid conjunctiva was cut out. The dye was extracted with formamide and subjected to absorbance determination at 625 nm. The therapeutic agent of the present invention (5 µl) was instilled into the eye used for induction 30 minutes and 1 hour prior to the passive anaphylaxis induction.

A predetermined amount of compound (I) was suspended in the base agent to be mentioned below to give compositions of the present invention as ophthalmic aqueous suspensions having a concentration of said compound of 0.01, 0.1, 0.3 or 1.0% (particle size 1.232 µm). As a control, physiological saline was used. The formulation of the base agent was as follows.

| | |
|---|---|
| Sodium chloride | 0.9 g |
| Polysorbate 80 | 0.1 g |
| Hydrochloric acid or phosphoric acid | appropriate amount |
| Sodium dihydrogenphosphate | 0.1 g |
| Distilled water | amount to make the total 100 ml (pH 7.0) |

The results of Experimental Example 1 are shown in Table 1 and Figure 1.

**Table 1**

| | Concentration (%) | n | Dye leakage (µg/site) | Suppression (%) |
|---|---|---|---|---|
| Physiological saline | - | 20 | 16.93±2.02 | - |
| Compound (I) | 0.01 | 8 | 13.16±3.95 | 22.3 |
| | 0.1 | 20 | 11.86±1.55 | 29.9 |
| | 0.3 | 20 | 10.46±1.57* | 38.2 |
| | 1.0 | 20 | 10.71±1.77* | 36.7 |
| Note : Each value is mean±S.E. | | | | |

| | | | | |
|---|---|---|---|---|
| * means significant difference from control, p<0.05. | | | | |

As is evident from Table 1, the control showed 16.93±2.02 µg/site of dye-leakage. The compositions of the present invention showed concentration-dependent suppressive effects, wherein they exhibited the suppression of 22.3%, 29.9%, 38.2% and 36.7% at concentrations of 0.01%, 0.1%, 0.3% and 1.0%, respectively, indicating significant difference at the concentrations of 0.3% and 1.0%. The composition of the present invention was effective against allergic conjunctivitis by instillation at the concentration of not less than 0.01%, particularly at not less than 0.3%.

### Experimental Example 2 : Irritation in anterior segment of an eye

In the same manner as in Experimental Example 1, the compositions of the present invention inclusive of 0.005%, 0.01% and 0.3% solutions (pH 6.9, osmotic pressure 299 mOsm), and 0.1% and 1.0% ophthalmic aqueous suspensions (pH 6.8 and 6.9, respectively, average particle size 1.4 µm) were obtained. Physiological saline was used as the control.

The therapeutic agents of the present invention were instilled into the left eye and control substance to the right eye of male Japanese white rabbits (weight 2.00 - 2.35 kg, 3 per group) by one drop (about 0.05 ml) per instillation, 8 times at one hour intervals. The anterior segment of the eye was visually observed before instillation and 30 minutes after 2nd, 4th, 6th and 8th instillations; flourescein-dyed spots in cornea were visually observed before instillation and 30 minutes after the final instillation; and eye behavior was observed upon each administration.

Visual observation of the anterior segment of the eye was performed with respect to changes in cornea, iris and conjunctiva (redness of palpebral conjunctiva, edema of palpebral conjunctiva, redness of bulbar conjunctiva, nictitating membrane and discharge), and there were found no abnormalities. Observation of the dyed spots in cornea revealed some increase in the dyed spots in the eye into which test substance was instilled. However, no significant difference was found from the eye administered with the control substance and the increase was within the normal range. With regard to the eye behavior, eye blinking and lid close were not found.

The results of Experimental Examples 1 and 2 suggest that the composition of the present invention had superior effects against allergic conjunctivitis by instillation and high safety level to the eye.

Preparations having the following formulation were prepared by a conventional method.

### Example 1 Eyedrop (ophthalmic aqueous suspension)

| | |
|---|---|
| Compound [I] | 1.0 g |
| Hydroxypropylmethylcellulose | 0.1 g |
| Boric acid | 2.0 g |
| Sodium hydroxide | appropriate amount |
| Distilled water | amount to make the total 100ml |

### Example 2 Eyedrop (ophthalmic aqueous suspension)

| | |
|---|---|
| Compound [I] | 2.0 g |
| Hydroxypropylmethylcellulose | 0.2 g |
| Boric acid | 2.5 g |
| Sodium edetate | 0.02 g |
| Sodium hydroxide | appropriate amount |
| Distilled water | amount to make the total 100ml |

### Example 3 Eyedrop (ophthalmic aqueous suspension)

| | |
|---|---|
| Compound [I] | 0.3 g |
| Polyvinyl alcohol | 0.2 g |
| Sodium acetate | 0.1 g |
| Propylene glycol | 2.0 g |
| Methyl p-hydroxybenzoate | 0.2 g |
| Propyl p-hydroxybenzoate | 0.1 g |
| Sodium hydroxide | appropriate amount |
| Distilled water | amount to make the total 100ml |

## Claims

1. Use of the amide compound of the formula (I) 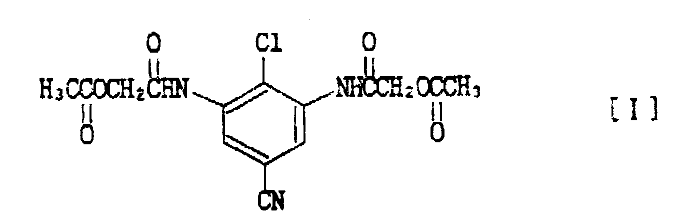 for preparing a pharmaceutical composition for topical administration to the eye for treatment of allergic conjunctivitis.

2. The use of Claim 1, wherein the pharmaceutical composition for topical administration to the eye for treatment of allergic conjunctivitis is in the form of an eyedrop.

3. The use of Claim 2, wherein the eyedrop comprises the amide compound of the formula (I) at a concentration of 0.001-2% (w/v).
